# EUROPEAN PATENT SPECIFICATION

(11) **EP 0 307 478 B1**
(45) Date of publication and mention of the grant of the patent: **27.09.1995**
(21) Application number: 88902211.7
(22) Date of filing: 26.02.1988
(51) Int. Cl.: C12N 9/64, C12N 15/00

(54) **SERINE PROTEASE AND SERINE PROTEASE GENE**
SERIN-PROTEASE UND SERIN-PROTEASE-GEN
PROTEASE DE SERINE ET GENE DE PROTEASE DE SERINE

(30) Priority: 05.03.1987 JP 50676/87; 09.09.1987 JP 225540/87
(43) Date of publication of application: 22.03.1989
(73) Proprietor: TORAY INDUSTRIES, INC., Tokyo 103 (JP)
(72) Inventor: AOKI, Yosuke, Tochigi-shi Tochigi-ken 328 (JP); OKANO, Kiyoshi, Kamakura-shi Kanagawa-ken 248 (JP); NARUTO, Masanobu, Kamakura-shi Kanagawa-ken 248 (JP); SHIMIZU, Hirohiko, Kamakura-shi Kanagawa-ken 248 (JP); NAKAMURA, Haruji, Hiratsuka-shi Kanagawa-ken 254 (JP)
(74) Representative: Kador & Partner
(86) International application number: JP8800205
(87) International publication number: WO8806621

(56) References cited:
- ANALYTICAL BIOCHEMISTRY, vol. 149, 1985, pages 153-162, Academic Press, Inc.;L.W. HECK et al.: "Isolation, characterization, and amino-terminal amino acidsequence analysis of human neutrophil elastase from normal donors"
- BIOL. CHEM. HOPPE-SEYLER, vol. 369, May 1988, pages 3-7, Walter de Gruyter& Co., Berlin, New York; D. FARLEY et al.: "Molecularcloning of human neutrophil elastase"
- THE JOURNAL OF BIOCHEMISTRY, vol. 102, no. 1, July 1987, pages 13-16, TheJapanese Biochemical Society; K. OKANO et al.: "Molecular cloning ofcomplementary DNA for human medullasin: an inflammatory serine protease in bonemarrow cells"
- NUCLEIC ACIDS RESEARCH, vol. 15, no. 22, 25th November 1987, pages 9601-9602,IRL Press, Ltd., Oxford, GB; H. NAKAMURA et al.: "Nucleotide sequence of humanbone marrow serine protease (medullasin) gene"
- The Journal of Biological Chemistry, Vol. 253, No. 6 (1978) Y. AOKI(Crystallization and Characterization of a New Protease in Mitochondria of BoneMarrow Cells). p. 2026-2032
- Biochemistry, Vol. 59, No. 10, October 1987 (Tokyo) Aoki Yosuke p. 1111-1133

## Description

### Technological Field

The present invention is concerned with a process for preparing a serine protease, a serine protease precursor and a process for preparing the same as well as with a serine protease precursor gene.

The serine protease is related to inflammation and is having an ability to change the functions of lymphocyte, monocyte, NK cell and granulocyte, and also relates to the gene coding the serine protease.

### Background Technology

Yosuke Aoki and others found a novel serine protease in erythroblast and granulocyte among myeloid cells and named the protease medullasin. They found that the the function of medullasin is to activate NK cells and to cause inflammation (J. Biol. Chem. 253, 2026-2032 (1978); J. Clin. Invest. 69, 1223-1230 (1982)).

Moreover, Yosuke Aoki and others found and reported that medullasin also had the below described biological activities.
(1) Medullasin took part in controlling heme synthesis in an erythroblast and manifestation of pyridoxine reactive anemia.
(2) Medullasin took part in manifestation of anemia accompanied with chronic inflammation.
(3) Medullasin activity in a granulocyte increased when chronic inflammatory diseases became worse.
(4) When medullasin of physiological concentration was injected inside of an animal skin, an inflammation characterized by monocyte infiltration was caused and endothelial cells of fine vein were characteristically denatured.
(5) when human lymphocytes were treated with medullasin, their ability of DNA and RNA syntheses increased and their reactivity to various mitogens remarkably increased.
(6) When monocytes were treated with medullasin, their migration ability was obstructed whereas their superoxide productivity was increased.
(7) When granulocytes were treated with medullasin, their migration ability was increased.
(8) When human lymphocytes were treated with medullasin, their NK activity was remarkably increased but that was not through interferon production.

The total amino acid sequence of medullasin had not been determined, and the only supplier of medullasin was human bone marrow cell or human granulocyte. So the amount of medullasin available was limited.

The first purpose of the present invention is to clone a gene corresponding to medullasin by means of a genetic recombination method to clarify the sequence. It is thereby possible to clarify the gene coding medullasin or its precursor and at the same time to elucidate the amino acid sequence of the medullasin. The second purpose of the present invention is to enable us to synthesize medullasin by means of a chemical synthesis or a genetic recombination, and to obtain medullasin with high purity in large quantities.

Medullasin is contained in human myeloid cell or peripheral blood cells in large quantities. For example, about 10 µg of medullasin was found in 1 ml of human peripheral blood. This medullasin is hardly found in other tissues and cells but expressed specifically in human blood cells, especially only in leukocytes and erythroblasts.

Therefore, it is expected that to clarify the expression mechanism of medullasin gene is to elucidate gene expression being specific to human leukocytes or erythroblasts which have not been clarified before.

In general, cell-specific gene expression is controlled by not only factors (trans factors) such as proteins being specific to the cells and so on, but also by the gene sequences existing around and in the said chromosomal genes. Therefore, it is thought that a DNA sequence of a transcription controlling region, which is necessary for gene expression being specific to leukocytes and erythroblasts among human blood cells, exists around or in the chromosomal gene of the above described serine protease, medullasin derived from human myeloid cells.

Therefore, the third purpose of the present invention is to propose a DNA sequence of a transcription controlling region being necessary for the cell-specific gene expression.

### Disclosure of the Invention

Object of the present invention is a process for preparing a serine protease as defined in claim 1.

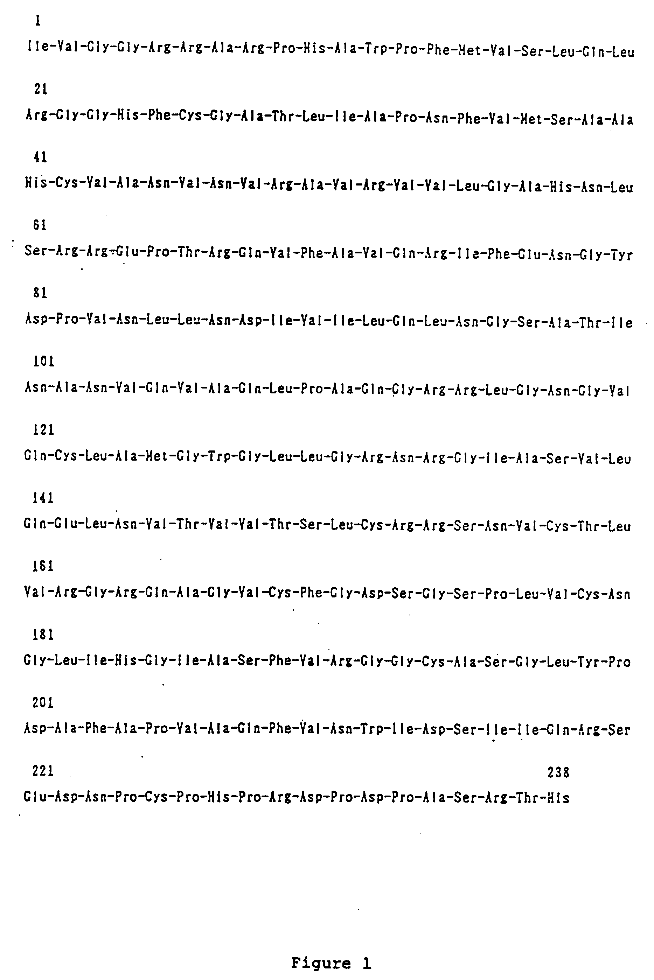

Moreover, the present invention is a serine protease precursor in which a cleavagable peptide or a signal peptide is connected to the N-terminal of the mature serine protease having the amino acid sequence shown in Figure 1 and the gene coding the serine protease precursor.

### Brief Explanations of Drawings

Figure 1, Figure 3 and Figure 5 show the partial or whole amino acid sequences of the serine protease of the present invention and Figure 2 and Figure 4 show the DNA sequences containing the serine protease gene of the present invention.

Figure 6 is an example of the amino acid sequence of the serine protease precursor of the present invention and Figure 7 shows an example of the DNA sequence containing the serine protease precursor of the present invention.

Figure 8 to Figure 14 show the method for preparing vectors expressing the serine protease of the present invention.

Figure 15 is an example of the DNA sequence coding the transcription controlling region contained in a chromosomal gene of the serine protease derived from human myeloid cells or granulocytes and Figure 16 shows the chromosomal gene sequence containing the serine protease structural gene and its expression controlling region of the present invention.

### The Best Embodiments for Practicing the Invention

The serine protease of the present invention is the polypeptide comprising the amino acid sequence of 238 residues as shown in Figure 1 and as far as the same biological activity as this is substantially being kept, those polypeptides constituted by partial substitution, deletion and insertion in the above described amino acid sequence are also included in the serine protease of the present invention.

It is known that in general, a protease loses a part of the N-terminal and moreover a part of the C-terminal in some cases by the fact that the precursor protein synthesized through a messenger RNA from its gene receives a processing and thereby becomes to the mature protease.

The present invention expresses the serine protease of those embodiments that for example an amino acid sequence of C-terminal 19 residues in a polypeptide constituted of the amino acid sequence shown in Figure 1 is deleted by receiving processing.

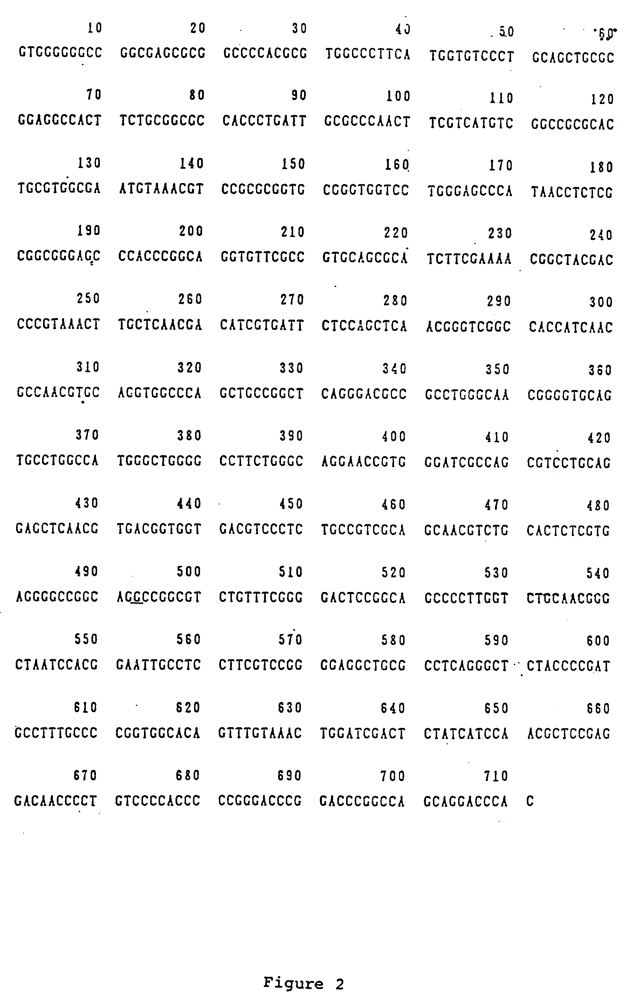

The serine protease gene of the present invention is the gene which codes the above described serine protease of the present invention and DNA sequence one containing the sequence shown in Figure 2 is one of the representative examples.

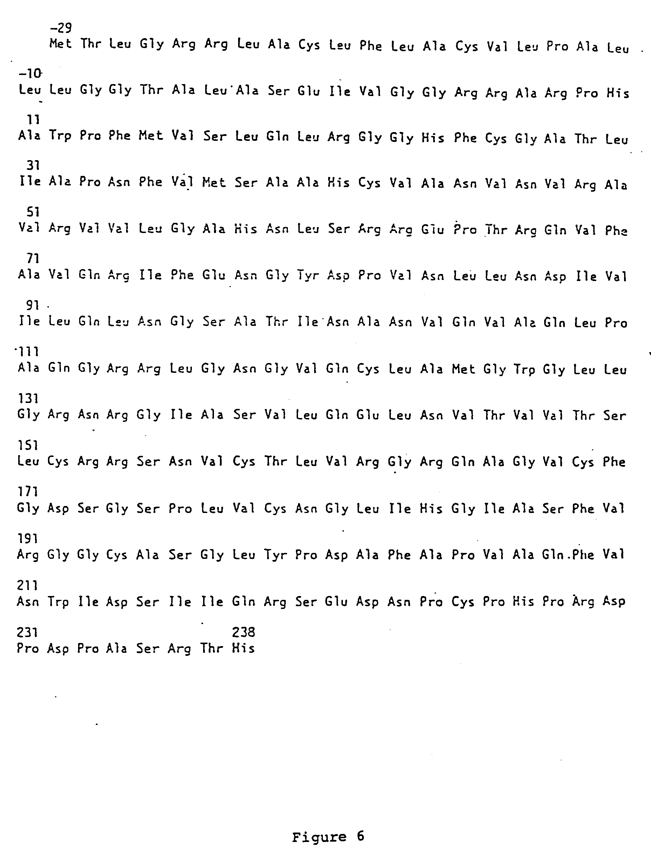

The serine protease precursor of the present invention is the one in which a cleavagable peptide or signal peptide is connected to the N-terminus of the above described serine protease and one of the examples is shown in Figure 6. The serine protease precursor shown in Figure 6 is constituted of the amino acid sequence of 267 residues and it is the one in which 29 amino acid sequence containing a signal peptide are connected to the upper stream of the serine protease shown in Figure 1.

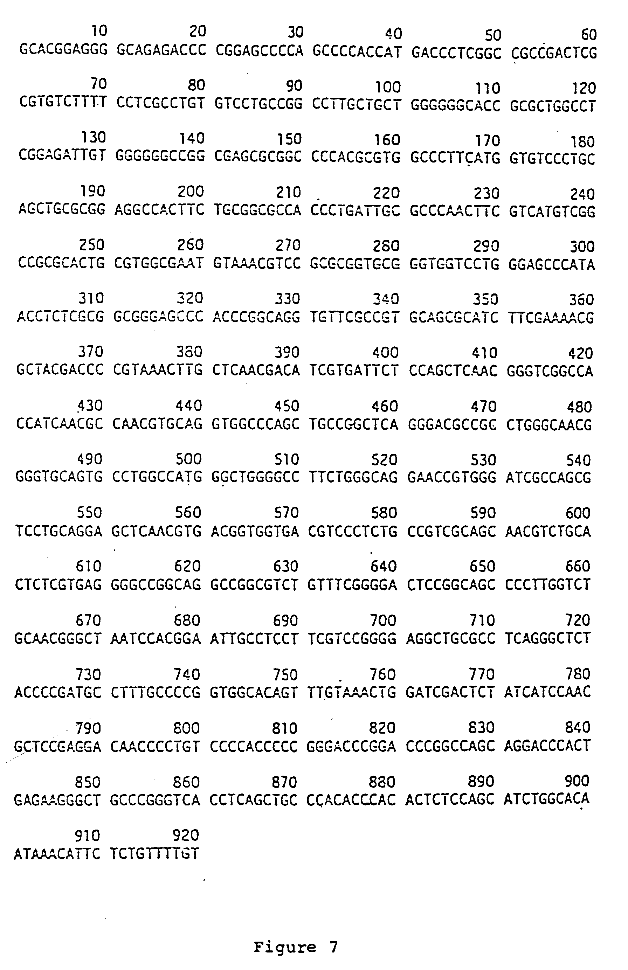

The serine protease precursor gene of the present invention is the gene coding the serine protease precursor and the one containing the DNA sequence shown in Figure 7 is its representative example.

As the whole amino acid sequence of the serine protease and its precursor of the present invention has been clarified by the present invention, it can be prepared by means of the known chemical synthesis, but also it can be prepared easily and in large quantities by means of a genetic recombination method.

For preparing the serine protease of the present invention by means of genetic recombination, at first, it is necessary to obtain the cDNA containing the serine protease gene. The cDNA can be preferably obtained by the following processes.
(1) Poly (A)⁺RNA is extracted from a cell producing the serine protease.
(2) cDNA is prepared by using the extracted Poly (A)⁺RNA as a template and reverse transcriptase, and a cDNA library containing various cDNAs is thereby obtained.
(3) A DNA probe hybridizing an aimed cDNA is chemically synthesized and the aimed cDNA is picked up by using this DNA probe from the cDNA library.

As the cell producing the serine protease used in the process (1), ML3 (cf. "Leukemia" edited by E. Henderson and F. Gunz, pp.119-139 (Grune & Strakton, New York, 1982)) which is the human APL (Acute Promyelocytic Leukemia) cell is preferably used because it continuously produces the serine protease.

The DNA probe used in the process (3) is obtained by chemically synthesizing and labelling a DNA oligomer which is a gene fragment corresponding to the amino acid sequence of the N terminal of the serine protease determined by Edman degradation method.

A vector containing the serine protease gene is integrated in an appropriate expression vector to obtain a recombinant DNA. The aimed serine protease can be produced by introducing this recombinant DNA into such a host as Escherichia coli, Bacillus subtilis, a yeast or a cultured animal cell and by culturing the transformed cells obtained thereby. If such a eucaryotic cell as an animal cell is used as a host in this process, serine protease having a sugar chain can be obtained.

The above described gene manipulation can be carried out by the known method (T. Maniatis et al. "Molecular Cloning, A Laboratory Manual" (Cold Spring Harbor Lab. 1982)).

Moreover, when Escherichia coli is used as a host, the expression efficiency can be improved by using an expression vector containing a gene coding a chimera protein constituted by connecting such protein as a peptide derived from T7 phage, an anthranilic acid synthesizing enzyme (abbreviated as TrpE hereinafter) or β-galactosidase as a removable form to the upper stream of the serine protease of the present invention.

The serine protease can be easily isolated by digesting the chimera protein prepared above with an enzyme and so on.

Moreover, the present invention relates to a DNA sequence coding a transcription controlling region contained in a chromosomal gene of serine protease derived from a human myeloid cell.

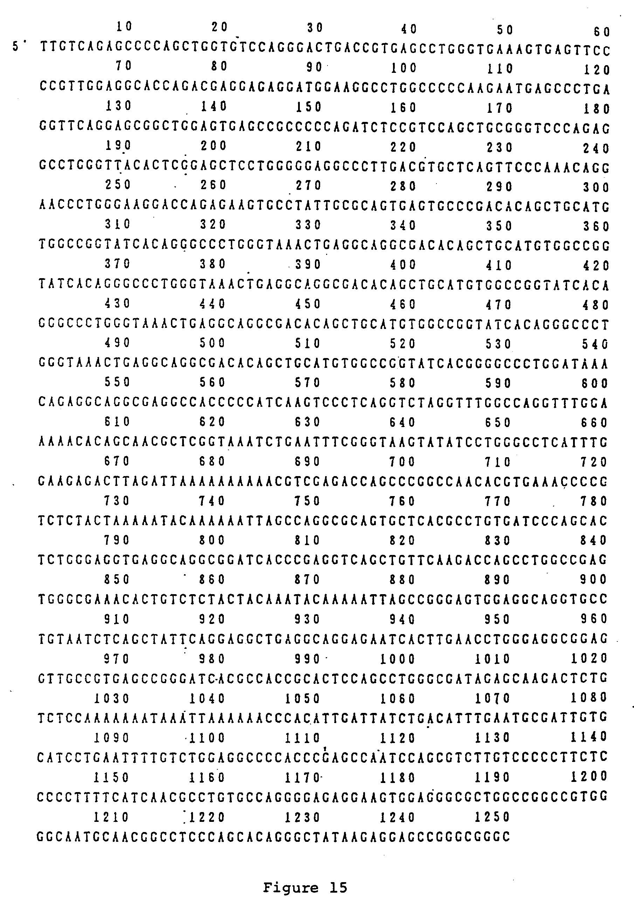

The transcription controlling region of the present invention contains a promoter region and an enhancer and as the DNA sequence coding them, for example, the sequence shown in Figure 15 or a sequence being equivalent thereto can be cited, but it is not restricted thereto. The word of "equivalence" means here those ones in which the DNA bases are partly substituted with other DNA bases, are partly eliminated or other DNA bases are added thereto and which at the same time have the same functionality as that of the original DNA sequence.

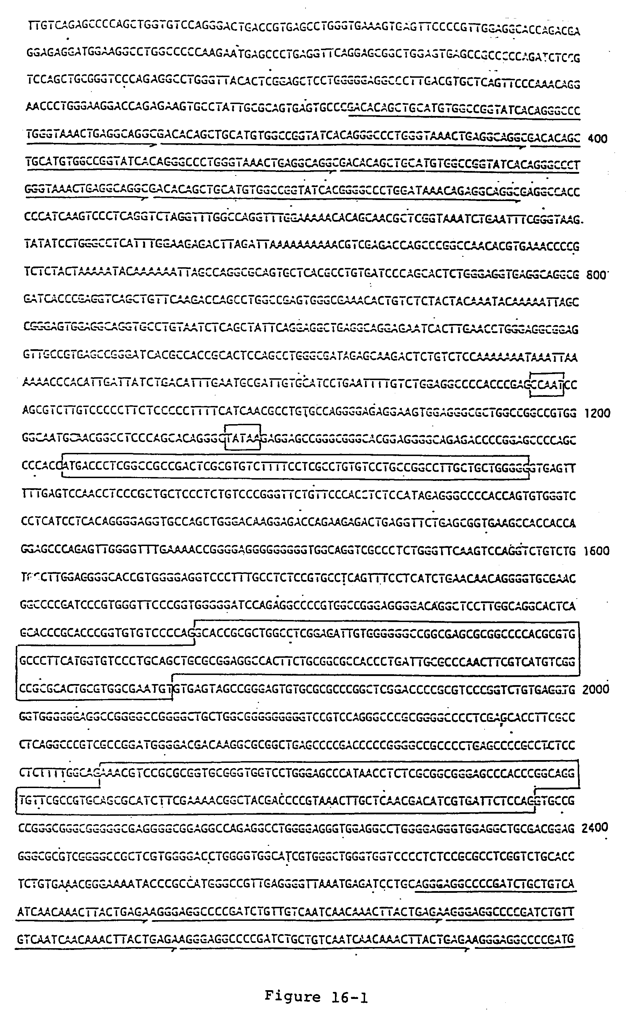

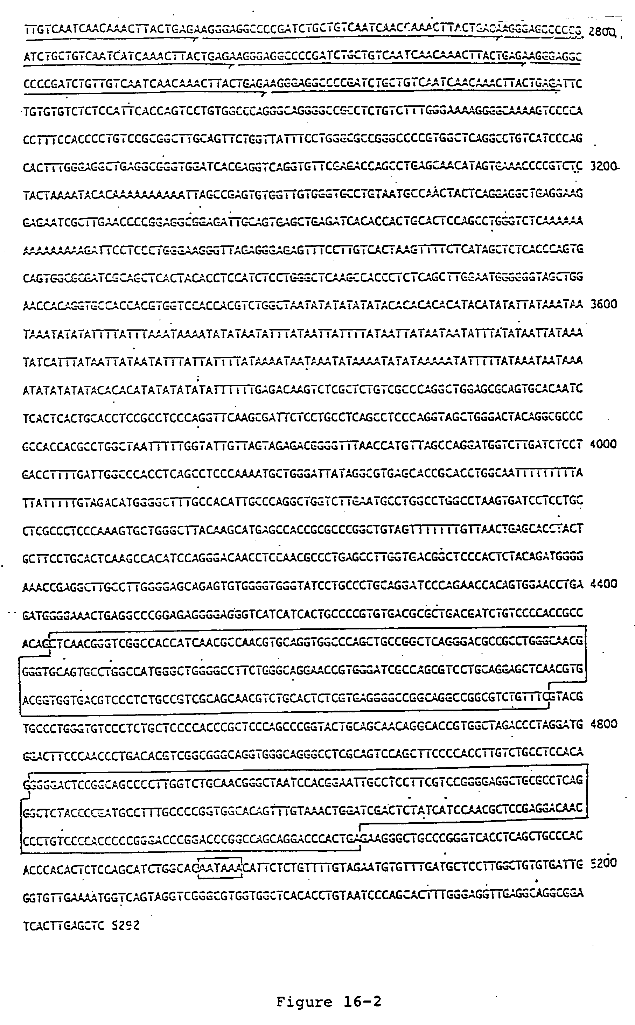

The sequence shown in Figure 15 corresponds to the 1st to 1,250th DNA sequence in the DNA sequence shown in Figure 16. Figure 16 shows a chromosomal gene sequence containing a structural gene a medullasin and its expression controlling region and this is the base sequence having been determined in a DNA fragment of about 6 kilobase having been cloned. In this sequence of Figure 16, the whole length of the human medullasin gene is contained and TATA sequence and CAAT sequence which are characteristic promoter components can be also confirmed. Comparing with the base sequence of medullasin cDNA, it was clarified that the medullasin gene was constituted of 5 exons divided by 4 introns.

As the characteristic structures, 4 repeat sequences comprising 53 base pairs upstream of the promoter structure and 10 direct repeat structures comprising 42 base pairs in the third intron can be observed. An AT rich structure being capable of having a complicated second structure also exists in the third intron. A few sequences similar to the consensus sequence (GGCGGG, CCCGCC) bound by a SP1 protein which is a transcription controlling factor also exists near the promotor region.

The base sequence in a translation starting region of the medullasin gene well coincides with a base sequence which M. Kozack proposed as a necessary one for starting an effective translation and it is estimated that this is one of the reasons why the medullasin protein exists in relatively large quantities such as about 10 µg per 1 ml of human peripheral blood.

Moreover, as shown in the examples, the present inventors found that by examining the quantity of poly(A)⁺RNA in cells by means of the Northern blotting analysis, poly(A)⁺RNA of medullasin was expressed in large quantities in ML3 cells (described above) which are human acute promyelocyte leukemia cells , but very little expressed, for example, in diploid fibroblasts derived from a human fetal pulmonary tissue or in cell strain MIAPaCa-2 cells derived from a human pancreatic malignant epithelial tumor.

It can be said from these results that the transcription controlling region is a sequence for a specific gene expression in human hemocyte cells, especially erythroblasts and granulocytes.

The present invention will be hereinafter more concretely explained by the following examples. In the examples, the word of mature serine protease was used not to mix up the serine protease of the present invention with a serine protease precursor.

### Example 1

### Preparation of serine protease cDNA and determination of the base sequence

### (A) Synthesis of DNA probe

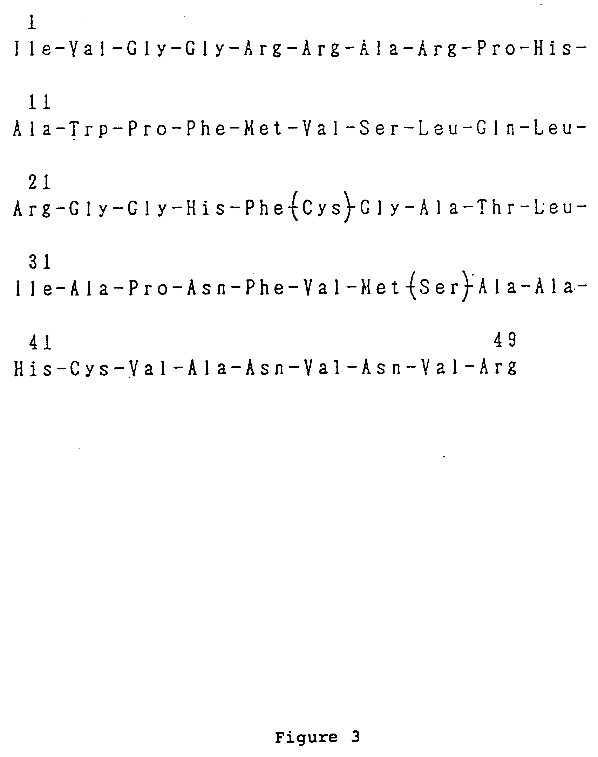

225 µg of purified medullasin obtained by means of the method of Aoki et al. (J. Biol. Chem. 253, 2026-2032 (1978)) were analyzed by using a gas phase automatic peptide sequencer type 470A (manufactured by Applied Biosystems Co., Ltd.). The sequence of 49 amino acid residues from the N terminal was determined as shown in Figure 3 by analyzing the obtained fraction by means of high-performance liquid chromatography. Some uncertainty was left by means of this analytical method in analyzing the amino acid residues being put in parentheses.

Among the above described 49 amino acid residues, a part of the amino acid sequence ¹²Trp-Pro-Phe-Met-¹⁶Val, in which the degree of degeneration of DNA to be coded was little, was selected and 8 kinds of DNA oligomers of 14 base length excluding a base of 5' terminal (this is the third base coding Val) having complementary DNA base sequence being complementary to DNA base sequences coding this part of the amino acid sequence were chemically synthesized by means of a known method. (It was based on the fact that there are 4 codons coding proline and 2 codons coding phenylalanine.) The base sequences of these 8 kinds of DNA oligomers were described as follows. 5' hydroxyl groups in the equivalent mixture of 8 kinds of DNA oligomers were labelled by phosphorylation with T4 polynucleotide kinase and γ ³²U.C.-ATP to obtain a DNA probe.

5'                         3'
A C C A T A A A A G G C C A
5'                         3'
A C C A T A A A C G G C C A
5'                         3'
A C C A T A A A G G G C C A
5'                         3'
A C C A T A A A T G G C C A
5'                         3'
A C C A T G A A A G G C C A
5'                         3'
A C C A T G A A C G G C C A
5'                         3'
A C C A T G A A G G G C C A
5'                         3'
A C C A T G A A T G G C C A

### (B) Preparation of Poly(A)⁺RNA

A cell line ML3 was cultured in RPMI 1640 medium containing 10% fetal colf serum at 37°C in 5% density carbon dioxide. When the cell reached about 1 x 10⁶ cells/mℓ, cycloheximide was added to the medium to make its concentration to be 30 µg/mℓ and the culture was continued for more 5 hours. 30 mℓ of a solution comprising 6M guanidine thiocyanate, 2% sarcosil, 50mM Tris hydrochloride (pH 7.6), 10mM EDTA and 1% β-mercaptoethanol was added to the cells obtained above (about 1.4 x 10⁹ cells) and a viscous solution obtained was passed through 18G injection needle five times.

This cell homogenate was placed on 5.7M cesium chloride solution (containing 100mM EDTA) whose volume was one third of the homogenate and centrifuged at 35000 rpm at 25°C overnight. RNA precipitated on the bottom of the centrifuge tube was dissolved in a small amount of water and precipitated with ethanol after phenol treatment to obtain 640 µg of total RNA.

This total RNA was fractionated in the usual way by means of oligo dT cellulose column chromatography to selectively obtain 86 µg of poly(A)⁺RNA.

### (C) Preparation of cDNA

Using 5 µg of poly(A)⁺RNA obtained in the preceding paragraph, 640 ng of double strand cDNA was synthesized by the method of Gubler and Hoffman (Gene, 25, 263(1983)) by utilizing a cDNA synthesizing kit (manufactured by Amersham) according to its protocol. Then, EcoRI linkers were connected to the both terminals by using the cDNA cloning system (manufactured by Amersham) and the reaction mixture was digested with EcoRI. 434 ng of double strand cDNA having EcoRI-attached terminals on its both ends were thereafter obtained by means of gel filtration column. 86 ng of this double strand cDNA was ligated to 1 µg of λgt10 arms by using T4 ligase and the reaction was added to the λ phage-packaging-extract and a mixture of recombinant phages (Materials of cDNA cloning system manufactured by Amersham Co., Ltd. and its recipe were used) was obtained. 9.6 x 10⁴ pfu (plaque-forming unit) of recombinant phages (cDNA library) were obtained by using Escherichia coli NM514 as a host.

### (D) Isolation of serine protease cDNA clone

About 25,000 recombinant phages obtained above were seeded on Escherichia coli NM514 as a host in one Petri dish whose diameter was 145 mm containing LB medium and 4 master plates were thereby prepared. After the phage was transferred on a nitrocellulose filter, phage DNA was fixed on the filter by means of alkaline denaturation. The procedure of R. Davis et al. ("DNA Cloning" edited by D.M. Glover, Vol. I, p.49 ∼ 78 (IRL Press) (1985)) was applied correspondingly to these procedures.

The filters where phage DNA was fixed by the above described procedure were screened by means of hybridization using the DNA probe prepared in paragraph (A). The hybridization was carried out at 35°C and washing was carried out at 35°C, too. The procedure of T. Maniatis ("Molecular Cloning, A Laboratory Manual" (Cold Spring Harbor Lab. (1982)) was applied correspondingly to these procedures. 7 positive of clones detected by means of autoradiography were obtained hybridization.

### (E) Determination of base sequence of serine protease cDNA

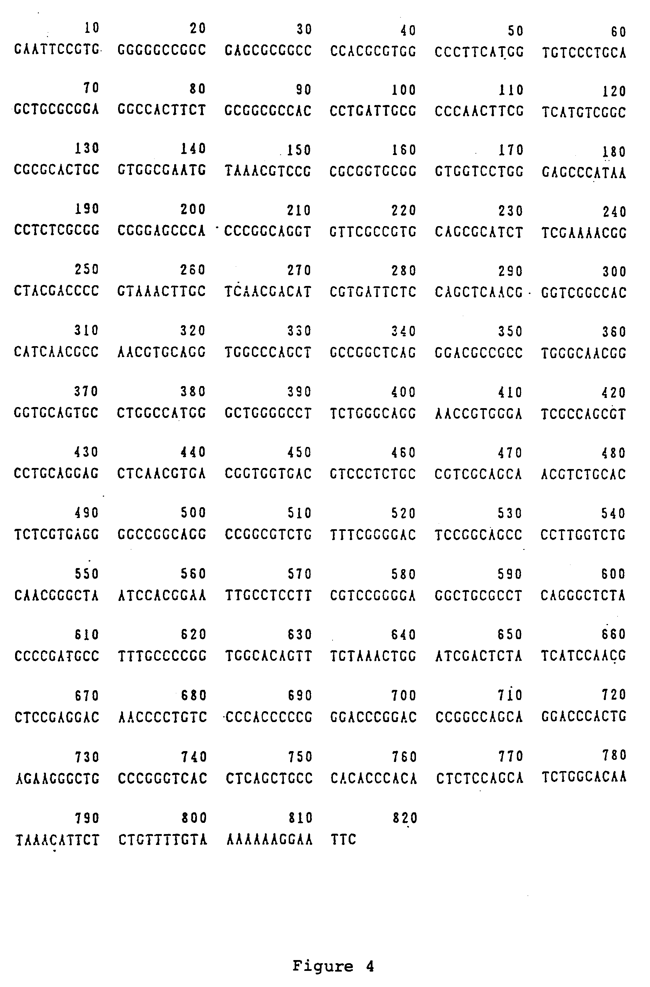

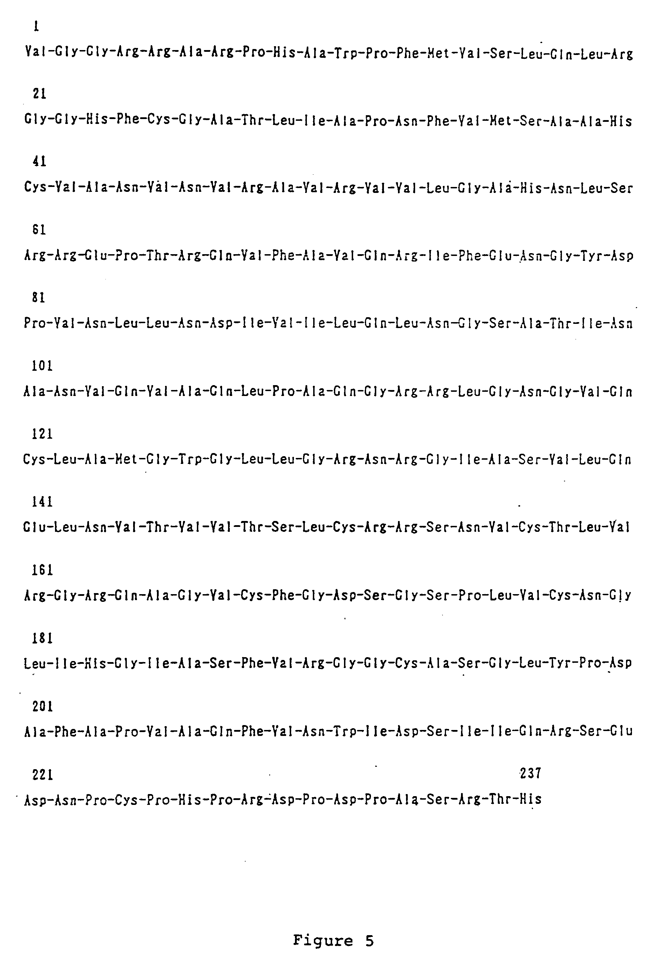

Phage DNA of MED1-4a which was one of the positive clones obtained in the preceding paragraph was extracted and cut by using EcoRI to obtain about 800 base pairs of DNA fragments. This fragment was inserted into EcoRI site of a cloning vector pUC19 (manufactured by Takara Shuzo Co., Ltd.), and of a phage M13mP19-RFDNA (manufactured by Takara Shuzo Co., Ltd.) for sequencing. Sequence analysis was carried out by means of the dideoxy method using deoxy-7-deaza guanine triphosphate. For analyzing the central parts, primers corresponding to the surrounding parts whose sequences had been already determined were successively synthesized and the sequence analyses were carried out by means of the dideoxy method. DNA sequence shown in Figure 4 was thus determined. In the DNA sequence shown in Figure 4, the upper stream including the 7th C and the lower stream including the 807th G were derived from EcoRI linker GGAATTCC used for cloning to λgt10 vector. Therefore, DNA base sequence from 8th to 806th in the figure was cDNA derived from poly(A)⁺RNA. The longest open leading frame (the protein-coding region) was searched and it was found that the translated coding frame of the DNA sequence from 8th to 718th in the figure was the longest and a termination codon TGA succeeded from the 719th one. The translated amino acid sequence corresponding to the DNA sequence from 8th to 718th was shown in Figure 5. The amino acid sequence from the 1st to the 48th in the figure completely identical with the amino acid sequence from the 2nd to the 49th of the purified medullasin shown in Figure 3. It be concluded from this result that the DNA sequence from the 8th to the 718th in the DNA sequence shown in Figure 4 constituted cDNA which codes the part of the serine protease of the present invention and this cDNA part was shown in Figure 2. The cDNA lacked the part coding isoleucine (Ile) which was the N terminal amino acid of purified medullasin shown in Figure 3, but because it was clear from the above described analysis of the amino acid sequence of the purified medullasin that Ile was the N terminal of medullasin, it can be concluded that whole cDNA sequence coding the serine protease of the present invention is the one where ATT, ATC or ATA coding Ile is added to the 5' terminal side of the base sequence shown in Figure 2. The amino acid sequence corresponding to this whole cDNA, namely, the amino acid sequence of the serine protease of the present invention was shown in Figure 1.

### Example 2

### Preparation of a full length cDNA of serine protease and determination of the base sequence

Five positive clones having longer serine protease cDNA than 800 base pairs of the clone MED1-4a obtained in Example 1 were isolated by using the partial cDNA obtained in Example 1 as a probe from about one million plaques of λgt10 cDNA library which was newly synthesized by the similar method as that of Example 1, paragraph (C) using 5 µg of ML3 cell poly(A)⁺RNA prepared in the similar way as Example 1, paragraph (B). The base sequence of the region coding N-terminals of serine protease of clone MED10 and MED13 among the five positive clones was determined by the same method as that of Example 1, paragraph (E) and it was found that MED13 and MED10 contained more 89 and 128 base pairs upper stream than that of MED1-4a. Therefore, the sequence of cDNA of serine protease precursor can be as shown in Figure 7. It was estimated from these DNA sequences that the serine protease precursor comprised 267 amino acid and more 29 amino acids were connected to the upper stream of isoleucine located at the N-terminal of a mature serine protease (Figure 6). Among the leader peptide comprising these 29 amino acids, 27 amino acids of the N-terminal side comprised 17 hydrophobic amino acid (9 leucines, 5 alanines, 1 phenylalanine, 1 valine and 1 proline) and had the most recognizable sequence with a signal peptidase comprising alanin-leucine-alanine on its C-terminal side. Therefore, they were thought to be a signal peptide. Moreover, it was estimated that a peptide comprising 2 amino acids, serine-glutamic acid, located on the lower stream of this leader peptide is an activation peptide.

### Example 3

### Preparation of a expression vector pETMED expressing serine protease in Escherichia coli and expression of T7-serine protease chimera protein

### (A) Preparation of a mature serine protease cDNA vector pUC19YMED:

At first the EcoRI fragment of cDNA obtained by Example 1 shown in Figure 4 was inserted in EcoRI site of a cloning vector pUC19 and a plasmid DNA pUC19MED1-4a was selected by confirming the inserting direction where the down stream part of the DNA shown in Figure 4 was close to BamHI site of pUC19.

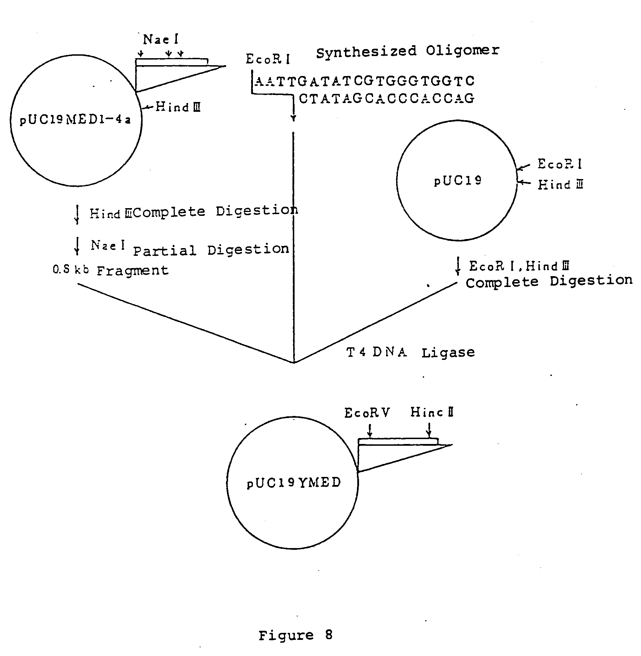

This vector pUC19MED1-4a was completely digested with HindIII and then partially digested with NaeI. An 810 base pair fragment of the serine protease cDNA was thereby obtained. A vector pUC19YMED having a mature serine protease cDNA was prepared by inserting this DNA fragment and a synthesized oligomer coding the N-terminal of the mature serine protease between EcoRI site and HindIII site of pUC19 by using T4 DNA ligase (Figure 8).

### (B) Preparation of pETMED:

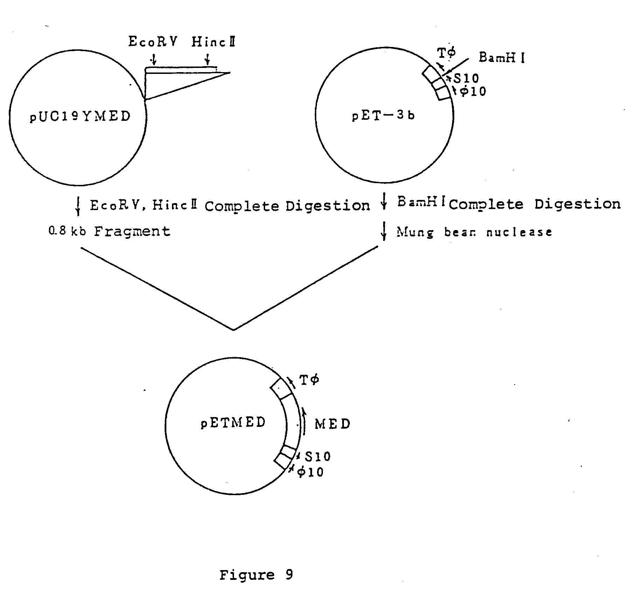

pET-36 distributed by Dr. F. William Studier (Biology Department, Brookhaven National Laboratory, Upton, New York) was completely digested with BamHI located on the lower stream of T7 promoter and thereafter made to a blunt end by Mung bean nuclease treatment. Then, pUC19YMED of the paragraph (A) was completely digested with EcoRV and HincII and about 0.8Kb of DNA fragments comprising a maturation serine protease cDNA were isolated. A vector pETMED expressing serine protease Escherichia coli was prepared by connecting these two DNA fragments by using T4DNA ligase (Figure 9). From this vector, a chimera protein having 250 amino acids where a peptide comprising 11 amino acids (Met-Ala-Ser-Met-Thr-Gly-Gly-Gln-Gln-Met-Gly) derived from T7 phage and a mature serine protease comprising 238 amino acids were connected each other between which arginine was placed, was prepared. The mature serine protease can be isolated by digesting partly this chimera protein with trypsin.

### (C) Expression of T7-serine protease chimera protein by using pETMED:

At first, pETMED prepared in the paragraph (B) was introduced into Escherichia coli HMS174 (J.L. Campbell et al., Proc. Natl. Acad., Sci., U.S.A. 75, 2276-2280 (1978)). This recombinant was cultured at 37°C overnight on 5 ml of LB medium (T. Maniatis et al., "Molecular Cloning", p.440 (1982)) containing 100 µg/ml of ampicillin and 0.4% (w/v) of maltose and then 0.1 ml of this was transferred on a NZCYM medium (T. Maniatis et al., "Molecular Cloning", p.440 (1982)) containing 100 µg/ml of ampicillin and 0.4% (w/v) of maltose. This was cultured at 37°C until the absorbance at 600 nm became to 0.3, thereafter infected with phage CE6 (F.W. Studier et al., J. Mol. Biol., 189, 113-130 (1986)) whose quantity was 5 to 10 times of Escherichia coli and cultured at 37°C for 3 hours. By analysing the whole protein of the bacteria obtained above by means of SDS polyacrylamide gel electrophoresis, a protein whose molecular weight was about 29,000 was observed and which was not observed in bacteria where a control pET-36 (above described) was introduced. It was confirmed by means of Western blotting using an anti-medullasine antibody that this protein was T7 serine protease chimera protein.

### Example 4

### Preparation of a vector pATH2MED expressing TrpE-serine protease and expression of TrpE-serine protease chimera protein

### (A) Preparation of a vector pATH2MED expressing TrpE-serine protease:

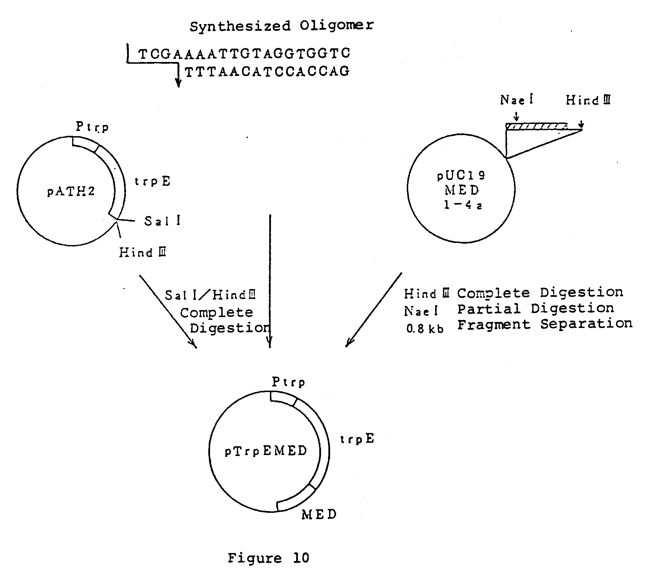

A DNA fragment of about 800 base pairs containing a serine protease partial gene was isolated by digesting completely pUC19MED1-4a in Example 1 with HindIII and thereafter digesting partially with NaeI. This fragment and a synthesized oligomer coding the N-terminal side of serine protease were inserted between Sa1I and HindIII sites of a vector pATH2 for expressing TrpE chimera protein (C.L. Dieckman and A. Tzagoloff, J. Biol. Chem. 260, 1513-1520 (1985)) by using T4 DNA ligase and thus a vector pATH2MED expressing a chimera protein of TrpE-serine protease was prepared (Figure 10).

A chimera protein comprising 570 amino acids in which a peptide comprising 331 amino acids derived from TrpE and a maturation serine protease comprising 238 amino acids were connected each other between which lysine was placed was prepared from this vector. The mature serine protease can be isolated by digesting partially this chimera protein with an endoprotease lysC.

### (B) Expressing TrpE-serine protease chimera protein by using pATH2MED:

Escherichia coli HB101 transformed with pATH2MED was cultured at 30°C for 7 hours in LB medium containing 100 µg/ml of ampicillin (T. Maniatis et al., "Molecular Cloning", p.440 (1982)). 4 ml of this culture was transferred to 40 ml of M9 medium (T. Maniatis et al. "Molecular Cloning", p.68 (1982)) containing magnesium sulfate, thiamin hydrochloride, glucose and ampicillin with the concentration of 1mM, 1 µg/ml, 1% (w/v) and 100 µg/ml respectively, cultured at 25°C overnight and further cultured for 8 hours after adding 0.8 ml of glucose, 0.16 ml of 14% (w/v) ammonium hydroxide and 40 µl of 10 mg/ml indoleacrylic acid.

By analyzing the whole protein of the bacteria by means of SDS polyacrylamide gel electrophoresis, a chimera protein of TrpE-serine protease whose molecular weight was about 61,000 and which was not observed in HB101 where a control pATH2 was introduced, was confirmed. It was confirmed by means of Western blotting using an anti-medullasin antibody that this protein was a chimera protein of the serine protease.

### Example 5

### Preparation of a serine protease yeast vector pATMED and Expression of a serine protease

### (A) Preparation of serine protease precursor cDNA vector pUC19PMED:

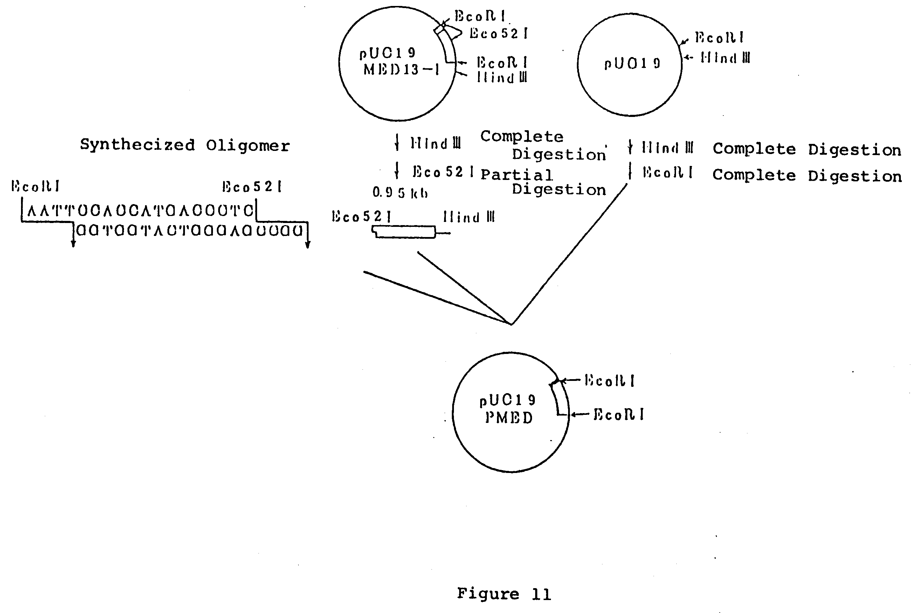

A DNA fragment of about 950 base pairs was separated by digesting completely pUC19MED13 in Example 3 with HindIII and thereafter digesting partially with Eco521. A vector pUC19PMED having a serine protease precursor cDNA was prepared by inserting this fragment and synthesized oligomers coding the N-terminal of serine protease precursor between EcoRI site and HindIII site of pUC19 by using T4 DNA ligase (Figure 11).

### (B) Preparation of pATMED:

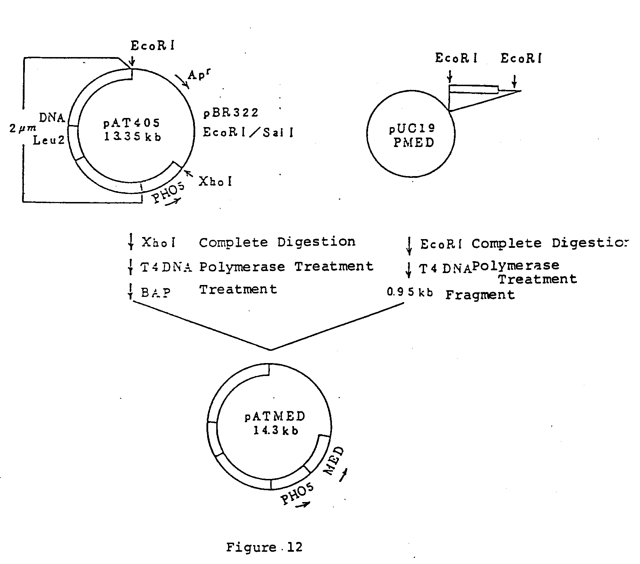

At first, a vector pAT405 having PH05 (inhibitory acidic phosphatase) promoter of a yeast distributed from Dr. Tobe of Hiroshima University was completely digested with XhoI and thereafter made to a blunt end by T4 DNA polymerase treatment. Phosphoric acid at of the terminal was removed by Escherichia coli alkaline phosphatase treatment. Then, pUC19PMED in the paragraph (A) was completely digested with EcoRI and thereafter made to blunt end by T4 DNA polymerase treatment. A DNA fragment of about 950 base pairs containing the serine protease precursor cDNA was thereafter isolated. A vector pATMED expressing a serine protease in yeast was prepared by connecting these 2 DNA fragments by using T4 DNA ligase (Figure 12).

### (C) Expressing a serine protease by using pATMED:

pATMED-introduced yeast DC5 (distributed by Dr. Tobe of Hiroshima University) was cultured at 30°C overnight in 5 ml of Yeast Nitrogen Base medium (manufactured by Difco) containing histidine and glucose with the concentration of 0.1 mg/ml and 2% (w/v) respectively. 2.5 ml of the culture was transferred to 50 ml of the same medium and cultured further at 30°C overnight. The bacteria were collected, washed once with 50 ml of water, suspended in 50 ml of a phosphorus-free medium (using KCl instead of KH₂PO₄ of Yeast Minimal Medium (R.L. Rodriguez and R.C. Trait " Recombinant DNA techniques" p151 (1983)) and cultivated at 30°C for a day and half. One ml of the cultured supernatant was concentrated by lyophilization and then analysed by SDS polyacrylamide gel electrophoresis. A protein whose molecular weight was about 32,000 and which was not observed in the cultured supernatant of DC5 in which a control pAT405 was introduced, was thereby detected. It was confirmed by means of Western blotting using an anti-serine protease antibody that this protein was the serine protease.

### Example 6

### Preparation of a vector pMFαMED expressing a yeast of serine protease and expression in a yeast

### (A) Preparation of a vector pMFαMED expressing a yeast of serine protease:

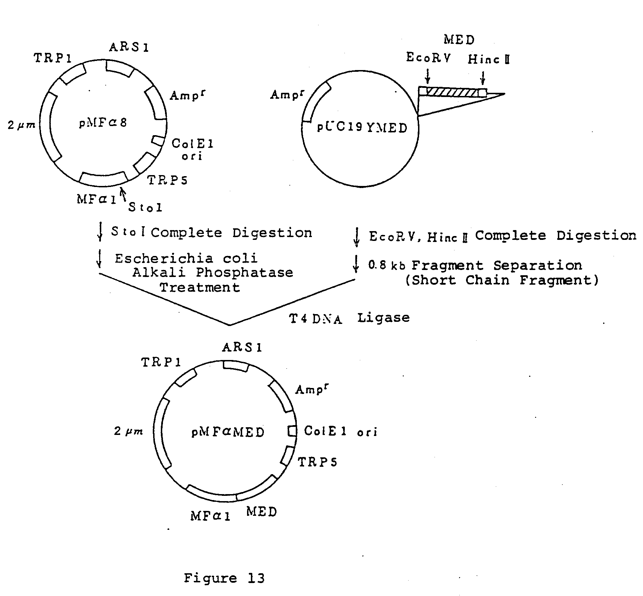

pUC19YMED in Example 4 paragraph (A) was completely digested with EcoRI and HincII and a DNA fragment of about 800 base pairs comprising a maturation serine protease cDNA was isolated. Then, a promoter of α-factor which was a mating pheromone of yeast and a vector pMFα8 (A. Miyajima et al., Gene, 37, 155 (1985)) having a leader sequence were completely digested with StuI and thereafter dephosphorylated by means of Escherichia coli alkaline phosphatase treatment. A vector pSRαMED for expressing the serine protease in yeast was prepared by connecting these two DNA fragments by using T4 DNA ligase (Figure 13).

### (B) Transformation of a yeast 20B12 with pSRαMED:

10 µg of pSRαMED in paragraph (A) were introduced into about 1 x 10⁷ cells of yeast 20B12 (distributed from Dr. Tobe of Hiroshima University) by means of alkaline metal treatment (A. Kimura et al., J. Bacteriol., 153, 163(1983)). The transformed cell thus obtained was cultured at 30°C overnight in 5 ml of a Yeast Nitrogen Base medium (manufactured by Difco) containing glucose with a concentration of 2% (w/v). 1 ml of the culture was then transferred to 10 ml of the same medium and cultivated for more 24 hours. 1 ml of the cultured supernatant was concentrated by lyophilization and then analyzed by means of Western blotting using an anti-serine protease (medullasin) antibody. A protein whose molecular weight was about 32,000 and which was not observed in the cultured supernatant of 20B12 in which a control pMFα8 was introduced, was thereby detected.

### Example 7

### Preparation of a vector pSRαMED expressing the serine protease in animal cell and expression in animal cell.

### (A) Preparation of a vector pSRαMED expressing the serine protease in animal cell:

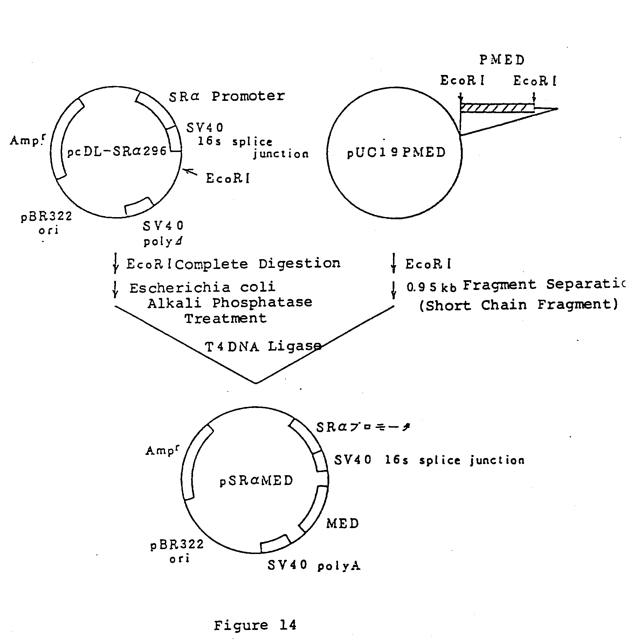

pUC19PMED in Example 6, paragraph (A) was completely digested with EcoRI and a DNA fragment of about 950 base pairs containing a serine protease precursor cDNA was separated. This was connected with a vector obtained by digesting pcDLSRα296 (obtained from Dr. Takebe, DNAX) completely with EcoRI and dephosphorylating it by means of Escherichia coli alkaline phosphatase treatment by using T4 DNA ligase and a vector pSRαMED expressing the serine protease in animal cell was thereby prepared (Figure 14).

### (B) Transformation of Cos-1 cell with pSRαMED:

10 µg pSRαMED in the paragraph (A) were introduced into about 2 x 10⁶ pieces of COS-1 cells derived from a monkey kidney (Y. Gluzman, Cell, 23, 175 (1981)) by means of calcium phosphate method (F.L. Graham et al., Virology, 54, 536). The cells were collected after 5 days and proteins of whole cells were analysed by means of Western blotting using an anti-serine protease (medullasin) antibody. A protein whose molecular weight was about 30,000 and which was not observed in COS-1 cells in which a vector was not introduced, was detected. Activity of the said enzyme was measured by the reported method (L. Visser et al., Biochem. Biophys. Acta, 268, 257 (1972)) using p-nitrophenyl N-tert-butyloxycarbonyl-L-alaninate which was a general substrate of elastases as the substrate. The soluble fraction obtained by breaking the cells by freeze-thaw and by centrifuging at 12,000 g for 30 minutes showed an activity increasing the absorbance at 347.5 nm by 0.1 per about 1 x 10⁶ cells in comparison with a control.

### Example 8

### Southern hybridization of chromosome DNA:

Before cloning of the human medullasin gene, at first, Southern hybridization was carried out. Each 10 µg of chromosome DNA derived from a human tonsil (The method of preparation was based on the method described in P. Chambon et al., Eur. J. Biochem, 36, p.32-38 (1973)) was cut with restriction enzyme EcoRI, BamHI ir PstI, fractionated using an agarose gel. Southern hybridization was carried out using medullasin cDNA as a probe after the Southern blotting. It was clarified from the result that only about 6 kb of fragment from EcoRI, digestion hybridized with the human medullasin cDNA probe and that only one kind of the human medullasin gene existed in the chromosome and it was contained in the 6 kb fragment of EcoRI.

The medullasin cDNA probe used here was prepared by labelling the cDNA (the EcoRI inserted fragment, about 800 bp) described in K. Okano et al., J. Biochem. 102, p.13-16 (1987) with ³²P by nick translation.

### Example 9

### Cloning of the human medullasin gene:

About 100 µg of human chromosome DNA was cut with EcoRI and the DNA of about 6 kb length was recovered from agarose gel electrophoresis. The recovered DNA was ligated with EcoRI digested arm-DNA of a vector λgtWES·λβ derived from a λ phage (obtained from BRL) as a vector (ligase reaction) and a gene library was prepared by means of in vitro packaging (The in vitro packaging kit was obtained from Takara Shuzo Co., Ltd.). A gene library of 5 x 10⁴ pfu could be prepared from 0.5 µg of the recovered DNA and 0.1 µg of the vector DNA. This gene library was screened by means of an ordinary plaque hybridization (T. Maniatis et al., "Molecular Cloning: A Laboratory Manual (abbreviated as a book A hereinafter)" p.312-318 (Cold Spring Harbor Laboratory (1982)) by using a nick translated medullasin cDNA as a probe (The nick translation kit was obtained from Takara Shuzo, Co., Ltd.) As the result, 3 pieces of positive clones were obtained. One of them was named as λMED-2.

### Example 10

### Determination of the base sequence of λMED-2:

The EcoRI inserted fragment of λMED-2 was sub-cloned in a sequencing vector pUC18 DNA or pUC19 DNA (obtained from Takara Shuzo Co., Ltd.) and appropriate deletion variants was obtained by using a kilo-deletion kit (obtained from Takara Shuzo Co., Ltd.) Each base sequence was determined by means of the dideoxy sequencing method using a 7-DEAZA sequence kit (obtained from Takara Shuzo Co., Ltd). The result was shown in Figure 1. Namely, among inserted fragments of cloned λMED-2, 5292 base sequence was determined and shown in Figure 1. CAAT box, TATA box and poly A signal were surrounded with small boxes and the regions coding proteins in medullasin mRNA were also surrounded with squares. Repeating sequences existing in the upper stream of a promoter and in the third intron were indicated with arrow-marked underlines. However, it is possible that the position of the 5' terminal of the mRNA a may be different from the actual position by some several bases.

### Example 11

ML3 cells, normal diploid fibroblasts derived from a human fetal lung and MIAPaCa-2 cells were cultured in 5% carbon dioxide gas at 32°C in a RPMI1640 medium containing a fetal calf serum (ML3), an Eagle's MEM medium containing fetal calf serum (normal diploid fibroblasts derived from a human fetal lung) or a Dalbecco's MEM medium containing 10% fetal calf serum and 2.5% equine serum (MIAPaCa-2 cells) respectively. 16 ml of a solution containing 6M guanidinethiocyanate, 2% sarcosil, 50mM (U.C.) Tris chloride (pH=7.6), 10mM EDTA, 10% β-mercaptoethanol were added in 2 to 5 x 10⁶ cells and the viscous solution obtained was passed through a 19G injection needle five times. This cell homogenate was placed on 18 ml of 5.7M secium chloride solution containing 100mM EDTA and centrifuged at 35,000 rpm at 25°C overnight. RNA precipitated on the bottom of the centrifuge tube was dissolved in a buffer solution and treated with phenol. Whole RNA was thereafter obtained by precipitating with ethanol. These procedures were carried out in accordance with the method described on page 196 of the book A. The quantity of the whole RNA thus obtained was 200 - 600 µg. These whole RNA were treated in an oligo dT column by the method described on page 192 - 198 of the book A to obtain a polyA(+)RNA. Each 10 µg of these polyA(+)RNA was fractionated with formaldehyde agarose gel electrophoresis and then transferred to a nitrocellulose filter. These procedures were carried out in accordance with the method described on page 202 - 203 of the book A.

The nitrocellulose filter thus obtained was analysed by means of the Northern hybridization, which was carried out in accordance with the Southern hybridization described in page 387 - 389 of the said book. As the probe, cDNA of serine protease (medullasin) described in the literature, K. Okano, et al., J. Biochem. 102, p.13-16 (1987) being labelled with [α³²P] dCTP by means of the translation (described on page 109 - 112 of the said book A) and whose specific activity was about 1 x 10⁸ cpm/µg, was used. The concentration of the probe was 2 x 10⁶ cpm/ml and the hybridization was carried out with the condition of 50% formamide at 42°C. By exposing a XAR-5 film of Kodak at -70°C overnight in the presence of an intensifying screen, a clear band was observed on the position of about 1,000 base length on the lane of RNA from ML3 cells. However, no detectable band was observed on the lanes where RNA obtained from other cells was electrophorased.

### Possibility of Industrial Application

Serine protease of the present invention takes part in manifestation of inflammation and it is therefore important to develop an anti-inflammatory drug. Moreover, it has an activity to change the functions of lymphocyte, monocyte, NK cell and granulocyte and therefore it is very useful in the medical care field.

Moreover, serine protease of the present invention can be useful medical supplies. For example, because serine protease of the present invention has a thrombus dissolving action, it is possible to use it as a thrombus dissolving agent for DIC (disseminated intravascular coagulation). Papain which is a protease derived from a plant may be used formerly in some cases for a medical treatment of DIC, it is anxious about that dangerous side effects may occur because the action is too strong and allergic reaction due to the antigenesity is induced. In this respect, there is a merit that serine protease of the present invention can be used safely because it is an enzyme derived from human being. Moreover, as another example of applications for medical supplies, for medical treatments of external injuries, it is possible to use the serine protease as a medicine for external application for removing and modifying granuloma-like rised tissues or old skin tissues. In this case, serine protease of the present invention can be safely used as it is derived from human being.

Moreover, the DNA sequence of the present invention of the transcription controlling region being necessary for the cell specific gene expression is useful for carrying out specifically the expression of foreign gene on a leukocyte or an erythroblast cell, above all, cultured cell strains derived from them.

## Claims

1. A process for preparing a serine protease which comprises culturing a transformed cell with an expression vector containing a serine protease gene coding the serine protease having the amino acid sequence shown in figure 1 and containing the DNA base sequence shown in figure 2.

2. A serine protease precursor where a cleavable peptide or signal peptide connects with the N-terminal of the serine protease having the amino acid sequence shown in figure 1.

3. A serine protease precursor according to claim 2 having the amino acid sequence shown in figure 6.

4. A serine protease precursor gene for coding the serine protease precursor of claim 3.

5. A serine protease precursor gene according to claim 4 containing the DNA base sequence shown in figure 7.

6. A process for preparing a serine protease precursor which comprises culturing a transformed cell with an expression vector containing a serine protease precursor gene according to claim 4.

## Patentansprüche

1. Verfahren zur Herstellung von Serin-Protease, das die Kultur einer transformierten Zelle mit einem Expressionsvektor umfaßt, der ein Serin-Protease-Gen enthält, das Serin-Protease codiert, das die in Figur 1 gezeigte Aminosäuresequenz hat und die in Figur 2 gezeigte DNA-Basensequenz enthält.

2. Serin-Protease-Vorstufe, wobei sich das abspaltbare Peptid oder Signalpeptid mit dem N-Terminus der Serin-Protease bindet, die die in Figur 1 gezeigte Aminosäuresequenz hat.

3. Serin-Protease-Präkursor nach Anspruch 2 mit der in Figur 6 gezeigten Aminosäuresequenz.

4. Serin-Protease-Präkursor-Gen zum Codieren des Serin-Protease-Präkursors nach Anspruch 3.

5. Serin-Protease-Präkursor-Gen nach Anspruch 4, das die in Figur 7 gezeigte DNA-Basensequenz enthält.

6. Verfahren zur Herstellung eines Serin-Protease-Präkursors, das die Züchtung einer transformierten Zelle mit einem Expressionsvektor umfaßt, der ein Serin-Protease-Präkursor-Gen nach Anspruch 4 umfaßt.

## Revendications

1. Procédé pour préparer une sérine-protéase, qui comprend la culture d'une cellule transformée avec un vecteur d'expression contenant un gène de sérine-protéase codant pour la sérine-protéase possédant la séquence d'acides aminés indiquée sur la figure 1 et contenant la séquence de base d'ADN indiquée sur la figure 2.

2. Précurseur de sérine-protéase dans lequel un peptide clivable ou un peptide signal est raccordé à l'extrémité N de la sérine-protéase possédant la séquence d'acides aminés indiquée sur la figure 1.

3. Précurseur de sérine-protéase selon la revendication 2, possédant la séquence d'acides aminés indiquée sur la figure 6.

4. Gène de précurseur de sérine-protéase codant pour le précurseur de sérine-protéase selon la revendication 3.

5. Gène de précurseur de sérine-protéase selon la revendication 4, contenant la séquence de base d'ADN indiquée sur la figure 7.

6. Procédé de préparation d'un précurseur de sérine-protéase qui comprend la culture d'une cellule transformée avec un vecteur d'expression contenant un gène de précurseur de sérine-protéase selon la revendication 4.
